# EUROPÄISCHE PATENTSCHRIFT

(11) **EP 2 819 631 B1**
(45) Veröffentlichungstag und Bekanntmachung des Hinweises auf die Patenterteilung: **30.03.2016**
(21) Anmeldenummer: 14718078.0
(22) Anmeldetag: 16.04.2014
(51) Int. Cl.: A61M 1/36, A61M 39/22

(54) **SPIKE MIT RÜCKSCHLAGVENTILFUNKTION SOWIE BEFÜLLVORRICHTUNG EINES FLÜSSIGKEITSSYSTEMS MIT SPIKE**
SPIKE WITH NON-RETURN VALVE FUNCTION AND FILLING DEVICE FOR A LIQUID SYSTEM, COMPRISING SAID SPIKE
PERFORATEUR DOTÉ D'UNE FONCTION CLAPET ANTIRETOUR ET DISPOSITIF DE REMPLISSAGE D'UN SYSTÈME DE LIQUIDE DOTÉ D'UN PERFORATEUR

(30) Priorität: 19.04.2013 DE 102013103986
(43) Veröffentlichungstag der Anmeldung: 07.01.2015
(73) Patentinhaber: B. Braun Avitum AG, 34212 Melsungen (DE)
(72) Erfinder: EIKELMANN, Guido, 34225 Baunatal (DE); HECTOR, Rainer, 49082 Osnabrück (DE)
(74) Vertreter: Winter, Brandl, Fürniss, Hübner, Röss, Kaiser, Polte - Partnerschaft mbB
(86) Internationale Anmeldenummer: PCT/EP2014/057745
(87) Internationale Veröffentlichungsnummer: WO 2014/170379

(56) Entgegenhaltungen:
- CA-A1- 2 807 013
- US-A- 3 827 601
- US-A- 5 334 315
- US-A- 5 540 653
- US-A1- 2011 208 128

## Beschreibung

Die vorliegende Erfindung betrifft einen Spike mit einer Rückschlagventilfunktion sowie die Befüllvorrichtung eines Fluidleitungssystems bzw. Flüssigkeitssystems, insbesondere eines extrakorporalen Blutbehandlungsgeräts beispielsweise einer Dialyse- oder Apheresemaschine, die mit dem Spike mit Rückschlagventilfunktion ausgerüstet ist.

### Hintergrund der Erfindung

Insbesondere das hydraulische System (blutseitiges Flüssigkeitssystem) eines Blutbehandlungsgeräts, beispielsweise einer Dialysemaschine muss vor Anschluss an einen Patienten mit einer Flüssigkeit, beispielsweise einer NaCl - Lösung oder einer anderen sterilen physiologischen Lösung, gefüllt werden, derart, dass Lufteinschlüsse in dem System entlüftet werden, die für einen, an das System flüssigkeits-angeschlossenen Patienten gefährlich sein würden. Des Weiteren sollte das hydraulische System mit der eingefüllten Flüssigkeit über einen bestimmten Zeitraum optional durchgespült werden, um im System möglicherweise abgelagerte Schadstoffe, Schmutzpartikel, etc. auszufiltern / auszuschwemmen, bevor das System am Patienten angelegt wird. Diese beiden Vorgänge werden bei einem extrakorporalen Blutbehandlungsgerät im Rahmen eines Befüll- und optional eines Zirkulationszyklus durchgeführt.

Ein entsprechend Konfiguriertes Blutbehandlungsgerät ist z.B. aus US 5 540 653 bekannt.

### Stand der Technik

Im Stand der Technik existieren Flüssigkeitsbehälter vorzugsweise in Form von Kunststoffbeuteln, die speziell für extrakorporale Blutbehandlungsgeräte dieser einschlägigen Gattung konstruiert sind, um die u. a. wie vorstehend definierten Gerätefunktionen zu ermöglichen. Solche Flüssigkeitsbehälter werden auch von der Anmelderin der vorliegenden Anmeldung hergestellt und beispielsweise unter der Bezeichnung "Ecobag" vertrieben.

Ein solcher Flüssigkeitsbehälter hat in der Regel eine Flüssigkeitsaufnahmekammer und zwei vorzugsweise verschließbare Flüssigkeitsanschlüsse. An einen ersten der zwei Anschlüsse ist ein arterieller Leitungsabschnitt und an den zweiten Anschluss ein venöser Leitungsabschnitt des hydraulischen Systems (Fluidsystems oder auch als Fluidleitungssystem bezeichnet) des extrakorporalen Blutbehandlungsgeräts anschließbar. Der Flüssigkeitsbeutel sowie die beiden Leitungsabschnitte bilden zusammen eine Zirkulationsvorrichtung des extrakorporalen Blutbehandlungsgeräts.

Für den Fluidsystem - Befüllvorgang wird gemäß einem Anmelder-internen Stand der Technik zunächst der arterielle Leitungsabschnitt an den ersten Flüssigkeitsanschluss des Beutels angeschlossen und nach Öffnen des ersten Flüssigkeitsanschlusses das hydraulische System befüllt. Dabei verbleibt der venöse Leitungsabschnitt des Systems zunächst zur Atmosphäre hin offen oder ist an einen Ablauf, ein Behältnis oder einen Beutel angeschlossen, sodass im System befindliche Luft zur Atmosphäre hin entweichen/entlüftet werden kann. Sobald der Befüllvorgang abgeschlossen ist, wird der venöse Leitungsabschnitt an den zweiten Flüssigkeitsanschluss des Beutels angeschlossen, um dann die im hydraulischen System des extrakorporalen Blutbehandlungsgeräts befindliche Flüssigkeit für eine bestimmte Zeitspanne oder um ein bestimmtes Strömungsvolumen über die Beutelkammer zu zirkulieren.

Bei diesem optionalen Zirkulationsvorgang durchströmt die Flüssigkeit systeminterne Filtereinrichtungen, in denen restliche Lufteinschlüsse mit der Flüssigkeit entfernt/ausgefiltert werden. Bei Bedarf kann der venöse Leitungsabschnitt des hydraulischen Systems wieder vom zweiten Flüssigkeitsanschluss des Fluidbeutels abgeklemmt und die im hydraulischen System befindliche Flüssigkeit unter ständiger Zufuhr von Flüssigkeit aus dem Behälter nochmals ausgespült werden.

Mit Beendigung des Zirkulationsvorgangs ist der eine Patientenbehandlung vorbereitende Befüll-/Zirkulationszyklus abgeschlossen, sodass die beiden Leitungsabschnitte (venös und arteriell) vom Flüssigkeitsbeutel abgetrennt und an den Patienten für eine Behandlung angelegt werden können.

Aus der vorstehenden Beschreibung des Befüll-/Zirkulationszyklus eines aus dem Anmelder- internen Stand der Technik bekannten hydraulischen Systems/Flüssigkeitsleitungssystems eines extrakorporalen Blutbehandlungsgeräts (Dialysemaschine) wird ersichtlich, dass für die Befüll- und Zirkulationsvorgänge der Fluidbeutel im Systemkreis verbleibt, d. h. die im System befindliche Flüssigkeit durch den Fluidbeutel bzw. durch dessen Fluidkammer zirkuliert wird. Damit kann möglicherweise die im Fluidbeutel bevorratete Flüssigkeit kontaminiert werden. Dies hat zur Folge, dass mit jeder neuen Behandlungsvorbereitung des extrakorporalen Blutbehandlungsgeräts ein neuer Fluidbeutel mit frischer, unkontaminierter Flüssigkeit für den folgenden Befüll-/Zirkulationszyklus eingesetzt wird, wohingegen der Fluidbeutel für den zuvor ausgeführten Befüll-/Zirkulationszyklus entsorgt wird, unabhängig von dessen Restfüllstand. Es liegt auf der Hand, dass bei dieser Vorgehensweise im Fall einer hohen Behandlungszahl ein mengenmäßig großer Teil an Flüssigkeit verschwendet wird, da der Flüssigkeitsinhalt eines Flüssigkeitsbeutels nur für einen Befüll-/Zirkulationszyklus (unvollständig) verwendbar ist.

Des Weiteren betreffen die Fluidbeutel für Blutbehandlungsgeräte beispielsweise auch die der Anmelderin selbst quasi eine Sonderanfertigung mit zwei separaten Fluidanschlüssen, wodurch sich aufgrund der gegenüber konventioneller NaCl - Beutel/Flaschen kleinen Stückzahlen die Fertigung insgesamt verteuert.

### Kurzbeschreibung der Erfindung

Angesichts dieser Problematik ist es die Aufgabe der vorliegenden Erfindung, eine technische Einrichtung insbesondere für eine gattungsgemäße Befüllvorrichtung eines Fluidleitungssystems vorzugsweise eines extrakorporalen Blutbehandlungsgeräts bereit zu stellen, mittels der die Kontamination eines daran angeschlossenen oder anschließbaren Fluidbeutels verhinderbar ist. Ein Ziel der vorliegenden Erfindung ist es ferner, eine Befüllvorrichtung sowie ein Befüllverfahren bereit zu stellen, was gegenüber dem Stand der Technik wirtschaftlicher und damit kostengünstiger betrieben werden kann. Des Weiteren ist ein Ziel der vorliegenden Erfindung, ein mit der erfindungsgemäßen Befüllvorrichtung ausgerüstetes Fluidsystem beispielsweise eines extrakorporalen Blutbehandlungsgeräts bereit zu stellen, das in einfacher Weise bedient werden kann und die Verwendung konventioneller Fluidflaschen (z.B. NaCl-Flaschen) erlaubt.

Diese Aufgabe wird durch eine Befüllvorrichtung eines Fluidsystems/Flüssigkeitssystems vorzugsweise eines extrakorporalen Blutbehandlungsgeräts (Dialyse-, Apheresemaschine) mit den Merkmalen des Anspruchs 1 gelöst. Ferner wird das weitere Ziel mit einem Fluidleitungssystem gemäß Anspruch 11 erreicht. Ferner wird die Aufgabe durch ein Verfahren mit den Merkmalen des Anspruchs 14 und/oder 15 gelöst. Vorteilhafte Ausgestaltungen der Erfindung sind Gegenstand der Unteransprüche.

Der Grundgedanke der vorliegenden Erfindung besteht darin, die gattungsgemäße Befüllvorrichtung eines Fluidleitungssystems insbesondere eines extrakorporalen Blutbehandlungsgeräts so auszubilden bzw. mit einer solchen Einrichtung auszurüsten, dass ein konventioneller (preisgünstiger) Fluidbehälter bekannter Bauart, etwa eine herkömmliche NaCl-Flasche oder ein entsprechender Beutel mit einem einzigen punktierbaren/ konnektierbaren Verschluss verwendet werden kann.

Erfindungsgemäß ist daher eine entsprechende Kontaminations-Verhinderungseinrichtung vorgesehen mit einem Spike für das Konnektieren eines Fluidbehälters (NaCl-Flasche/Beutel) sowie einer manuell betätigbaren Fluid-Sperreinrichtung für das wahlweise Fluidverbinden des Fluidbehälters mit einem zu befüllenden Fluidleitungssystem, welches an die Fluidsperreinrichtung anschließbar ist. Die Fluidsperreinrichtung ist demnach unmittelbar stromab zu dem Spike angeordnet ist, wobei die Fluidsperreinrichtung direkt mit dem Spike gekoppelt sein kann oder zwischen der Fluidsperreinrichtung und dem Spike ein Verbindungs-Schlauchstück zwischengefügt sein kann. Zusätzlich ist erfindungsgemäß ein Rückschlagventil zwischen Spike und Fluid-Sperreinrichtung vorgesehen, das dazu angepasst ist, nur eine Strömung vom Spike in Richtung zur manuell betätigbaren Fluid-Sperreinrichtung zuzulassen.

Des Weiteren hat die erfindungsgemäße Befüllvorrichtung als zentrale Bauelemente den sogenannten Spike für das Konnektieren des einzigen Fluidanschlusses eines konventionellen medizinischen Fluidbehälters (z.B. NaCl-Flasche), an den sich stromab die manuell betätigbare Fluid-Sperreinrichtung, etwa ein Sperrventil anschließt, die dazu angepasst oder dafür vorgesehen ist, fortdauernd mit dem Spike fluidverbunden zu bleiben. Die Fluid-Sperreinrichtung hat zumindest einen Fluid-Auslassanschluss, der dafür angepasst ist, dass ein Leitungsabschnitt/Schlauch eines Fluidleitungssystems/Flüssigkeitssystems vorzugsweise der arterielle Leitungsabschnitt eines Blutreinigungsgeräts/Dialysemaschine daran lösbar anschließbar ist. Das Rückschlagventil kann hierbei im Spike-Auslass, im Verbindungs-Schlauchstück oder im Einlass der manuell betätigbaren Fluid-Sperreinrichtung angeordnet sein, an den der Spike angeschlossen ist.

Durch die Anordnung der Fluid-Sperreinrichtung am Spike kann der Spike an dem konventionellen medizinischen Fluidbehälter nach Punktieren des Behälterverschlusses verbleiben, wohingegen der arterielle Leitungsabschnitt wahlweise an die Fluid-Sperreinrichtung für einen Befüllvorgang des Fluidleitungssystems anschließbar und danach wieder von der Fluid-Sperreinrichtung abklemmbar ist, ohne dass Fluid aus dem medizinischen Fluidbehälter verloren geht. Da der arterielle Leitungsabschnitt von dem Auslassanschluss der Fluid-Sperreinrichtung (z.B. Luer-Lock-Anschluss) abgekoppelt wird, kann der arterielle Leitungsabschnitt unmittelbar danach an einen arteriellen Patientenzugang wieder angeschossen werden, ohne dass am arteriellen Leitungsabschnitt bzw. dessen Anschluss irgend welche Veränderungen vorgenommen werden müssen. Dadurch wird die Handhabung der Befüllvorrichtung vereinfacht.

Das Rückschlagventil verhindert ferner zuverlässig eine Strömungsumkehr in den Fluidbehälter unabhängig von der Betätigungsstellung der Fluid-Sperreinrichtung. D.h., selbst wenn die Fluid-Sperreinrichtung unbeabsichtigt oder fehlerhaft betätigt wird oder eine Fehlfunktion aufweist, stellt das Rückschlagventil eine "Fail-safe" - Einrichtung dar.

Die Fluid-Sperreinrichtung ist eine 3 - Wege - Weiche, beispielsweise ein Y-oder T- Stück oder ein 3 - Wege - Hahn, wobei die Wege/Anschlüsse wahlweise manuell absperrbar sind (beispielsweise über Klemmen an den Wegen/Anschlüssen oder über den Hahn), um so die Wege vollständig zu verschließen und/oder die Wege wahlweise miteinander Fluid zu koppeln bzw. eine Fluidströmung zumindest zwischen zwei ausgewählten Wegen zuzulassen. Erfindungsgemäß ist die 3 - Wege - Weiche, vorzugsweise der 3 - Wege - Hahn unmittelbar diesem einen vorzugsweise universellen medizinischen Flüssigkeitsbehälter nachgeordnet. Im Konkreten wird ein erster Anschluss der 3 - Wege - Weiche (des 3 - Wege - Hahns) mit dem sogenannten Spike (oder einem andersartigen Anschlussmittel) gekoppelt oder ist mit diesem ausgebildet, mittels dem eine Fluidkammer des Flüssigkeitsbehälters angezapft werden kann. An einen zweiten Anschluss der 3 - Wege - Weiche (des 3 - Wege - Hahns) ist der arterielle Leitungsabschnitt und an einen dritten Anschluss der 3 - Wege - Weiche (des 3 - Wege - Hahns) ist ein venöser Leitungsabschnitt des Fluidsystems anschließbar. Die 3 - Wege - Weiche, vorzugsweise der 3 - Wege - Hahn ist weiter vorzugsweise manuell in wenigstens drei Stellungen bringbar bzw. schaltbar, von denen in einer ersten Schaltstellung der erste Anschluss ausschließlich mit dem zweiten Anschluss fluidverbunden bzw. eine Fluidströmung zwischen diesen beiden Anschlüssen zugelassen ist und der dritte Anschluss gesperrt ist (Spülstellung bzw. Zulauf - Schalterstellung bzw. Single-Pass-Schaltstellung), in einer zweiten Schaltstellung der zweite Anschluss mit dem dritten Anschluss fluidverbunden bzw. eine Fluidströmung zwischen diesen beiden Anschlüssen zugelassen ist und der erste Anschluss gesperrt ist (Zirkulation - Schaltstellung) und in einer dritten Schaltstellung alle drei Anschlüsse voneinander getrennt bzw. gesperrt sind (Therapie - Schaltstellung oder geschlossenen Schalterstellung).

Durch ein derart ausgerüstetes medizinisches Fluid-/Flüssigkeitssystem ist es möglich, den herkömmlichen Flüssigkeitsbehälter für den Befüllvorgang an den arteriellen Leitungsabschnitt zu koppeln (bei unangeschlossenem venösen Leitungsabschnitt), indem die 3 - Wege - Weiche, vorzugsweise der 3 - Wege - Hahn in die erste Schaltstellung gebracht wird, und den arteriellen Leitungsabschnitt mit dem venösen Leitungsabschnitt für den Zirkulationsvorgang zu verbinden, indem die 3 - Wege - Weiche, vorzugsweise der 3 - Wege - Hahn in die zweiten Schaltstellung gebracht wird. Da in dieser zweiten Schaltstellung der erste Anschluss der 3 - Wege - Weiche, vorzugsweise des 3 - Wege - Hahns gesperrt ist, wird der Flüssigkeitsbehälter vom hydraulischen System/Kreis des extrakorporalen Blutbehandlungsgeräts getrennt, sodass die darin sich befindende Restflüssigkeit nicht von der im hydraulischen System zirkulierenden Flüssigkeit kontaminiert wird. Da in der dritten Schaltstellung der 3 - Wege - Weiche, vorzugsweise des 3 - Wege - Hahns dessen sämtliche Anschlüsse gesperrt sind, können in dieser Schaltstellung die beiden Leitungsabschnitte des hydraulischen Systems abgeklemmt und an den Patienten für eine Behandlung angelegt werden.

Der Flüssigkeitsbehälter kann somit für mehrere Behandlungsabschnitte oder für mehrere Phasen innerhalb einer Behandlung eingesetzt werden in Abhängigkeit von dessen Füllvolumens, sodass keine Flüssigkeit verloren geht. Des Weiteren ist die medizinische Befüllvorrichtung für einen konventionellen/universellen medizinischen Flüssigkeitsbehälter vorgesehen, der gegenüber den für extrakorporale Blutbehandlungsgeräte speziell angefertigten Behältern mit zwei Anschlüssen preisgünstiger ist. Schließlich braucht der verwendete Flüssigkeitsbehälter gar keinen Anschluss insbesondere für den Fall, dass an die 3 - Wege - Weiche, vorzugsweise den 3 - Wege - Hahn der sogenannte Spike angeschlossen oder mit diesem zu einer integralen Baueinheit zusammengefasst ist. Im letzteren Fall kann der Spike quasi übergangslos, d.h. ohne Zwischenschaltung eines (überbrückenden) Leitungsstücks unmittelbar mit dem ersten Anschluss vorzugsweise einstückig verbunden oder mit diesem verschraubt sein.

Das erfindungsgemäße medizinische Fluidsystem/Fluidleitungssystem vorzugsweise eines Blutreinigungsgeräts (Dialysemaschine) hat eine Befüllvorrichtung gemäß vorstehend beschriebenem Aufbau sowie einen venösen und einen arteriellen Blutleitungsabschnitt, von denen zumindest der arterielle Leitungsabschnitt dafür angepasst ist, wahlweise an die Befüllvorrichtung angeschlossen zu sein/zu werden. Des Weiteren hat zumindest der arterielle Leitungsabschnitt unmittelbar stromab zu seinem mit der Befüllvorrichtung zusammenwirkenden Anschluss (ein Bauelement) eine Sperreinrichtung (z.B. Schlauchklemme als weiteres Bauelement)), mittels welcher der arterielle Leitungsabschnitt temporär für ein Umklemmen von der Befülleinrichtung zu einem arteriellen Patientenzugang und umgekehrt fluidgesperrt werden kann. Vorzugsweise weist auch der venöse Leitungsabschnitt die gleichen Bauelemente auf wie der arterielle Leitungsabschnitt.

Die Erfindung wird nachstehend anhand eines bevorzugten Ausführungsbeispiels unter Bezugnahme auf die begleitenden Figuren näher erläutert.
Fig. 1 zeigt ein Fluidsystem vorzugsweise in Form einer Zirkulationsvorrichtung eines extrakorporalen Blutbehandlungsgeräts gemäß einem bevorzugten Ausführungsbeispiel der Erfindung,
Fig. 2 zeigt eine "geschlossene Schaltstellung" einer Befüllvorrichtung in Form eines 3 - Wege - Hahns der Zirkulationsvorrichtung gemäß Fig. 1 als eine mögliche Variante der erfindungsgemäßen Befüllvorrichtung (hier 3 - Wege - Weiche), wobei an dieser Stelle bereits darauf hingewiesen wird, dass beispielsweise auch ein Y- oder T-Stück mit Schlauchsperrmittel (Schlauchklemmen) an jedem Zweigweg stromauf zu den Befüllvorrichtungs-internen Anschlüssen vorgesehen sein kann,
Fig. 3 zeigt eine "Spülstellung" des 3 - Wege - Hahns der Zirkulationsvorrichtung gemäß Fig. 1, in der eine Flüssigkeitsquelle (Flüssigkeitsbehälter) ausschließlich mit einem arteriellen Leitungsabschnitt des extrakorporalen Blutbehandlungsgeräts fluidverbunden ist,
Fig. 4 zeigt eine "Zirkulation - Schaltstellung" des 3 - Wege - Hahns der Zirkulationsvorrichtung gemäß Fig. 1, in welcher der arterielle Leitungsabschnitt ausschließlich mit einem venösen Leitungsabschnitt des extrakorporalen Blutbehandlungsgeräts fluidverbunden (kurzgeschlossen) ist und die Flüssigkeitsquelle vom extrakorporalen Blutbehandlungsgerät fluidgetrennt ist,
Fig. 5 zeigt eine andere "geschlossene Schaltstellung" eines 3 - Wege - Hahns der Zirkulationsvorrichtung gemäß Fig. 1 und
Fig. 6a-6d zeigen symbolisch die Kontaminations-Verhinderungseinrichtung der Befüllvorrichtung an unterschiedlichen Positionen zwischen dem Spike und der manuell betätigbaren Absperreinrichtung vorzugsweise in Form des 3 - Wege - Hahns.

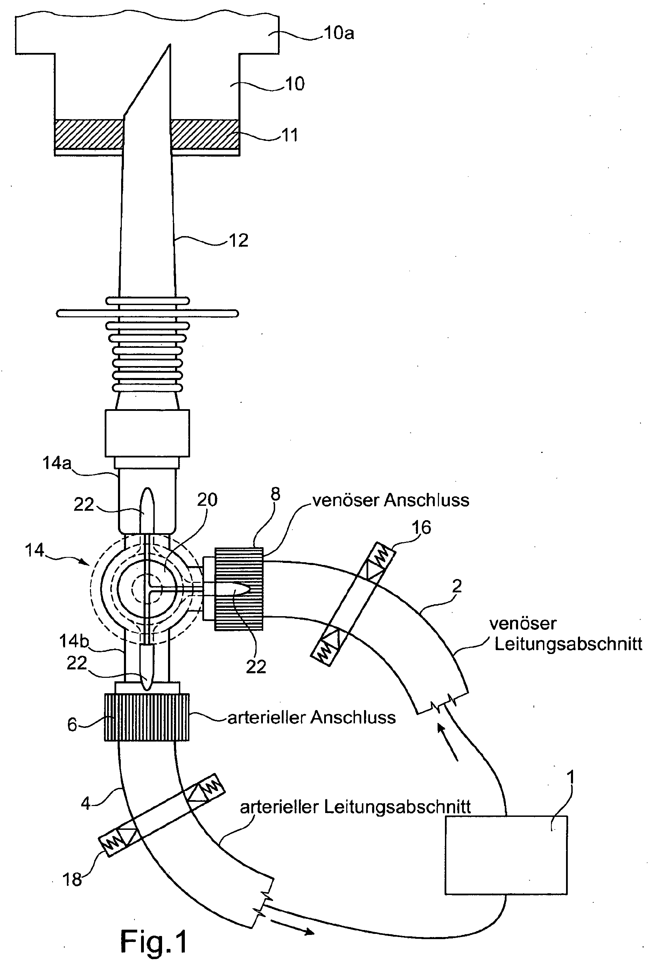

Gemäß der Fig. 1 hat ein extrakorporales Blutbehandlungsgerät 1, beispielsweise eine Dialyse- oder Apheresemaschine ein internes hydraulisches Leitungssystem (nachfolgend als Fluidleitungssystem bezeichnet), durch das während einer Behandlungsphase auf der Maschinenseite beispielsweise eine Blut - Reinigungsflüsssigkeit (Dialysierflüssigkeit) geleitet wird und auf der Patientenseite Blut extrakorporal hindurchfließt, wobei das maschinenseitige und patientenseitige Fluidleitungssystem im Fall einer Dialysemaschine durch einen nicht weiter dargestellten Dialysator (Filter) fluidisch getrennt sind.

Hierfür hat das Fluidleitungssystem patientenseitig einen venösen sowie einen arteriellen Leitungsabschnitt 2, 4, vorzugsweise jeweils Schlauchabschnitt mit jeweils endseitig angeordneten/ ausgebildeten Anschlüssen (Luer-Lock-Anschlüssen) 6, 8, an die beispielsweise Injektionsnadeln oder Kanülen (nicht dargestellt) als Patientenzugang anschließbar sind, welche in einen Patientenkörper einführbar sind.

Um ein möglicherweise notwendiges Ausschwemmen von ggf. herstellungsbedingten Verunreinigungen in den Patientenkörper zu vermeiden, hat das extrakorporale Blutbehandlungsgerät 1 eine Befüllvorrichtung , welche vorliegend einen Zirkulationsprozess ermöglicht (daher nachfolgend als Zirkulationsvorrichtung bezeichnet), mittels welcher das Fluidleitungssystem in der Regel vor jeder Patientenbehandlung gereinigt wird.

Gemäß der Fig. 1 hat die Befüll-/Zirkulationsvorrichtung gemäß der vorliegenden Erfindung eine Flüssigkeitsquelle vorzugsweise in Form eines universellen Flüssigkeitsbehälters (NaCl-Flasche) 10 mit einem einzigen Auslass 11, der beim vorliegenden Ausführungsbeispiel mittels eines Spike 12 der Befüllvorrichtung punktiert wird, um Flüssigkeit aus dem Flüssigkeitsbehälter 10 abzuzapfen. Der Aufbau des Spike 12 entspricht bekannten Spikekonstruktionen, sodass an dieser Stelle auf dessen Aufbau nicht weiter eingegangen werden muss. Ferner sei darauf hingewiesen, dass beispielsweise im Fall eines Luer-Lock- oder andersartigen Anschlusses auf der Behälterseite der Spike durch ein entsprechendes Anschlussstück auf Seiten der Befüll-/Zirkulationsvorrichtung ersetzt sein kann.

Des Weiteren hat die erfindungsgemäße Befüll-/Zirkulationsvorrichtung eine Fluidsperreinrichtung vorliegend in Form einer 3 - Wege - Weiche, vorzugsweise einen 3 - Wege - Hahn 14, der dem Spike 12 (Anschlussstück) in Strömungsrichtung weg vom Flüssigkeitsbehälter 10 gesehen, unmittelbar nachgeschaltet ist. Im vorliegenden Fall ist der Spike 12 direkt (ohne Zwischenschaltung eines zusätzlichen Leitungsstücks) an den 3 - Wege - Hahn 14 angeschlossen. Alternativ kann der Spike 12 auch einstückig bzw. als eine Baueinheit mit dem 3 - Wege - Hahn 14 ausgebildet sein.

Der 3 - Wege - Hahn 14 hat hierfür einen ersten Anschluss oder Fluideinlass 14a, der mit dem Spike 12 fluidverbunden ist, bzw. an den die Flüssigkeitsquelle 10 anschließbar/angeschlossen ist. Des Weiteren hat der 3 - Wege - Hahn 14 einen zweiten Anschluss 14b, an den der arterielle Leitungsabschnitt 4 des patientenseitigen Fluidleitungssystems des extrakorporalen Blutbehandlungsgeräts 1 anschließbar ist. Schließlich hat der 3 - Wege - Hahn 14 einen dritten Anschluss 14c, an den der venöse Leitungsabschnitt 2 des patientenseitigen Fluidleitungssystems des extrakorporalen Blutbehandlungsgeräts 1 anschließbar ist. Sowohl der venöse wie auch der arterielle Leitungsabschnitt 2, 4 ist jeweils mit einer Schlauchklemme 16, 18 oder dergleichen Sperrmittel versehen, um den zugehörigen Schlauchabschnitt wahlweise temporär zu verschließen.

Der 3 - Wege - Hahn 14 hat vorliegend einen manuell betätigbaren Drehverschluss bestehend aus einem drehbaren zylindrischen Ventilkolben 20, der stirnseitig mit einer Handhabe vorzugsweise in Form, von (drei) Eingriffsflügeln 22 versehen/ausgebildet ist. Der Ventilkolben hat eine zentrale Längsbohrung von der drei in Umfangsrichtung gleichmäßig beabstandete Radialbohrungen abzweigen. Die Eingriffsflügel 22 sind so angeordnet, dass sie sich längs der Radialbohrungen ausrichten und so die Strömungsrichtung der Radialbohrungen anzeigen. Ein solcher 3 - Wege - Hahn ist aus dem Stand der Technik hinlänglich bekannt, sodass an dieser Stelle auf eine weitere Beschreibung insbesondere von dessen Funktion verzichtet werden kann.

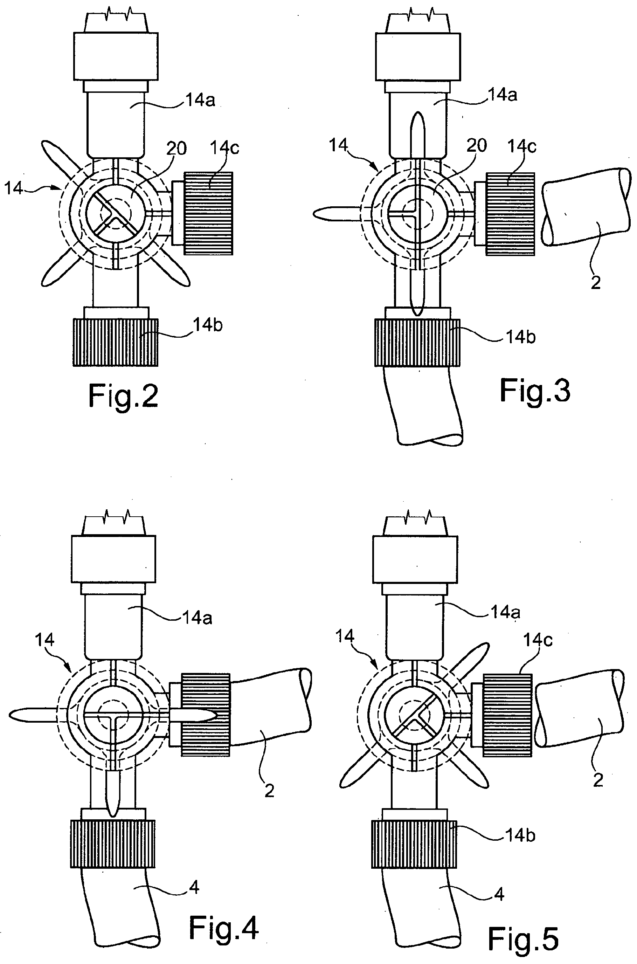

In den Fig. 2 bis 5 sind die erfindungsgemäß beabsichtigten Schaltstellungen des 3 - Wege - Hahns 14 in Abhängigkeit von den aktuellen Betriebsphasen des extrakorporalen Blutbehandlungsgeräts 1 dargestellt, welche nachfolgend in Zusammenhang mit den dadurch bezweckten Funktionen beschrieben werden.

Gemäß der Fig. 2 ist der 3 - Wege - Hahn 14 in einer Sperrposition gezeigt, in der alle drei Anschlüsse 14a - 14c geschlossen sind. In dieser Schaltstellung kann der Spike 12 den Auslass 12 des Universal - Flüssigkeitsbehälters 10 punktieren und somit die darin (in der vom Behälter ausgebildeten Kammer 10a ) gespeicherte Flüssigkeit anzapfen, ohne dass Flüssigkeit zur Atmosphäre hin verloren geht.

Die Fig. 3 zeigt die so genannte "Spülstellung", in welcher der erste Anschluss 14a mit dem zweiten Anschluss 14b fluidverbunden ist, wohingegen der dritte Anschluss 14c gesperrt wird. In dieser Schaltstellung ist bereits der arterielle Leitungsabschnitt 4 an den zweiten Anschluss 14b angeschlossen, wobei jedoch der venöse Leitungsabschnitt 2 zur Atmosphäre hin offen ist oder an einen Abfluss/Sammelbehälter angeschlossen ist.

In dieser Schaltposition wird Flüssigkeit (NaCl-Lösung) aus dem konventionellen Flüssigkeitsbehälter mit einem einzigen Anschluss über den 3 - Wege - Hahn 14 in den arteriellen Leitungsabschnitt 4 geleitet und dadurch das patientenseitige Fluidleitungssystem fortlaufend geflutet, solange, bis die Flüssigkeit aus dem venösen Leitungsabschnitt 2 herausläuft oder in einen Abfluss/Sammelbehälter hineinläuft. solange, bis die Flüssigkeit aus dem venösen Leitungsabschnitt 2 herausläuft. D.h. während dieses System-Füllvorgangs dient der venöse Leitungsabschnitt 2 als Entlüftung. Dabei sei darauf hingewiesen, dass während dieses Vorgangs die Schlauchklemmen 16, 18 natürlich geöffnet sind.

Sobald das System mit Flüssigkeit aus dem Flüssigkeitsbehälter 10 aufgefüllt ist, wird der venöse Leitungsabschnitt an den dritten Anschluss 14c des 3 - Wege - Hahns 14 angeschlossen und der 3 - Wege - Hahn 14 in die Schaltstellung gemäß der Fig. 4 verstellt, welche als "Zirkulation" Schaltstellung bezeichnet werden kann. In dieser Schaltstellung sind der zweite und der dritte Anschluss 14b, 14c des 3 - Wege - Hahns 14 miteinander fluidverbunden, wohingegen der erste Anschluss 14a gesperrt wird.

Wird nunmehr die im Fluidleitungssystem befindliche Flüssigkeit zirkuliert, durchströmt diese ausgehend vom venösen Leitungsabschnitt 2 den 3 - Wege - Hahn 14 und wird von dort wieder in den arteriellen Leitungsabschnitt 4 weiter geleitet, ohne dass Flüssigkeit in den Flüssigkeitsbehälter 10 eindringen kann. Die darin gespeicherte Flüssigkeit bleibt somit unkontaminiert.

Nach einer vorbestimmten Zirkulationsdauer wird der 3 - Wege - Hahn 14 in die in Fig. 5 gezeigte Schaltstellung weitergeschaltet, in welcher erneut alle drei Anschlüsse 14a - 14c gesperrt sind. Es ist zu erkennen, dass sich die Schaltstellung gemäß der Fig. 5 von der Schaltstellung gemäß der Fig. 2 unterscheidet, da der Ventilkolben 20 nicht einfach in die erste Sperrstellung gemäß Fig. 2 voll - rückgedreht wurde, sondern in die zweite Sperrstellung gemäß der Fig. 5 teil - rückgedreht wurde, welche demnach der ersten Sperrstellung diametral gegenüberliegt. Wäre der Ventilkolben 20 nämlich voll - rückgedreht worden, hätte er zumindest die "Spülstellung" temporär durchlaufen, in der möglicherweise kontaminierte Flüssigkeit aus dem Fluidleitungssystem in den Flüssigkeitsbehälter hätte eindringen können.

Sobald der 3 - Wege - Hahn 14 gesperrt ist, wird nunmehr der venöse Leitungsabschnitt 2 erneut vom 3 - Wege - Hahn 14 abgeklemmt und der Ventilkolben 20 in die Spülstellung gemäß Fig. 3 gedreht, umein erneutes Spülen nach der Zirkulation durchzuführen. Mit Beendigung dieses Vorgangs ist der Befüll-/Zirkulationszyklus abgeschlossen.

Abschließend wird der 3 - Wege - Hahn 14 erneut gesperrt, alle Schlauchklemmen 16, 18 stromab zum 3 - Wege - Hahn 14 in Sperrstellung gebracht und auch der arterielle Leitungsabschnitt vom 3 - Wege - Hahn 14 abgeklemmt, um zusammen mit dem venösen Leitungsabschnitt am Patientenkörper angelegt werden zu können.

An dieser Stelle sei darauf hingewiesen, dass gemäß der vorstehenden Beschreibung die erfindungsgemäße Befüllvorrichtung mit der 3 - Wege - Weiche als Fluid - Sperrvorrichtung ausgebildet ist, um einen Zirkulationsmodus ausführen zu können. Es ist aber auch denkbar, die erfindungsgemäße Sperrvorrichtung als einfaches Schließventil mit einem Ein- und einem Auslassanschluss auszubilden, welches dann nur einen "Spülbetrieb" gemäß vorstehender Definition zulässt. In jedem Fall erlaubt die Befüllvorrichtung gemäß der vorliegenden Erfindung ein vorzugsweise manuelles Sperren das Spike - Auslasses, sodass der Spike nach Abschluss des "Spülbetriebs" im Flüssigkeitsbehälter verbleiben und zumindest der hiervon abgeklemmte arterielle Leitungsabschnitt über dessen internen Anschluss an den bereits gelegten Patientenzugang ohne weitere Maßnahme angeschlossen werden kann.

Ferner ist es denkbar, eine weitere (oder alternativ zur voll geschlossenen Schaltposition) Schaltposition für die 3 - Wege - Weiche vorzusehen, in der alle drei Wege geöffnet und damit miteinander fluidverbunden sind. Diese Position hat dann technische Relevanz, wenn beispielsweise sterile Produkte verwendet werden.

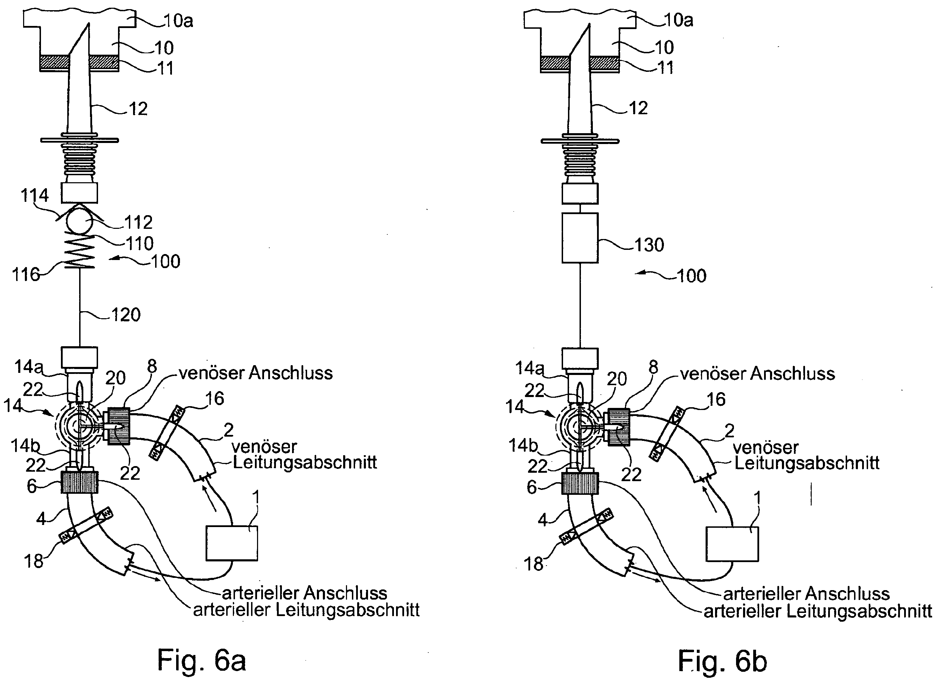

In den Fig. 6a-6d ist die Befüllvorrichtung der vorhergehenden Figuren insbesondere im Bereich der erfindungsgemäßen Kontaminations-Verhinderungseinrichtung 100 dargestellt.

Demzufolge ist zwischen dem Spike 12 und dem einen Anschluss (Einlass) 14a des 3 - Wege - Hahns 14 ein Rückschlagventil 110 vorgesehen, das eine Fluidströmung vom Spike 12 in Richtung zum 3 -Wege - Hahn 14 zulässt und eine Fluidströmung in die Gegenrichtung blockiert. Das Rückschlagventil 110 hat einen Ventilkörper 112, der mittels einer Feder 116 gegen einen Ventilsitz 114 vorgespannt ist, um diesen entgegen der Fluidströmung vom Spike 12 kommend zu schließen.

Die Fig. 6a-6d zeigen dabei verschiedene Varianten für die Positionierung des Rückschlagventils 110.

Gemäß der Fig. 6a ist das Rückschlagventil 110 unmittelbar mit dem Spike 12 gekoppelt und zwar ohne Zwischenfügen eines Verbindungsschlauchs. Beispielsweise könnte das Rückschlagventil 110 in diesem Fall direkt an den Auslassanschluss des Spike 12 angeschossen oder sogar in diesen integriert sein. Ferner könnte in diesem Fall zwischen dem Rückschlagventil 110 und dem 3 -Wege - Hahn 14 vorzugsweise ein Verbindungsschlauchelement 120 vorbestimmter Länge angeordnet werden, wobei die Länge so gewählt ist, dass ein Schlauchvolumen entsteht, welches ausreicht, eine gewisse Menge an rückströmendem Fluid aufzunehmen, bevor dieses das Rückschlagventil 110 bzw. den Spike erreicht. D. h. es wird durch das Verbindungsschlauchelement 120 eine Art Pufferspeicher gebildet, die zu einer verbesserten Kontaminationsverhinderung beiträgt, insbesondere dann, wenn das Rückschlagventil 110 fehlerhaft ist und nicht vollständig schließt.

Gemäß der Fig. 6b ist in dem Verbindungsschlauchelement 120 zwischen dem Spike 12 und dem 3 -Wege - Hahn 14 eine Tropfkammer 130 zwischengefügt, welche mit einer Rückschlagventilfunktion versehen ist. Die Tropfkammer 130 kann hierbei ebenfalls unmittelbar am Spike 12 montiert sein, wobei das nachgeschaltete Verbindungsschlauchelement 120 den vorstehend beschriebenen Pufferspeicher bildet.

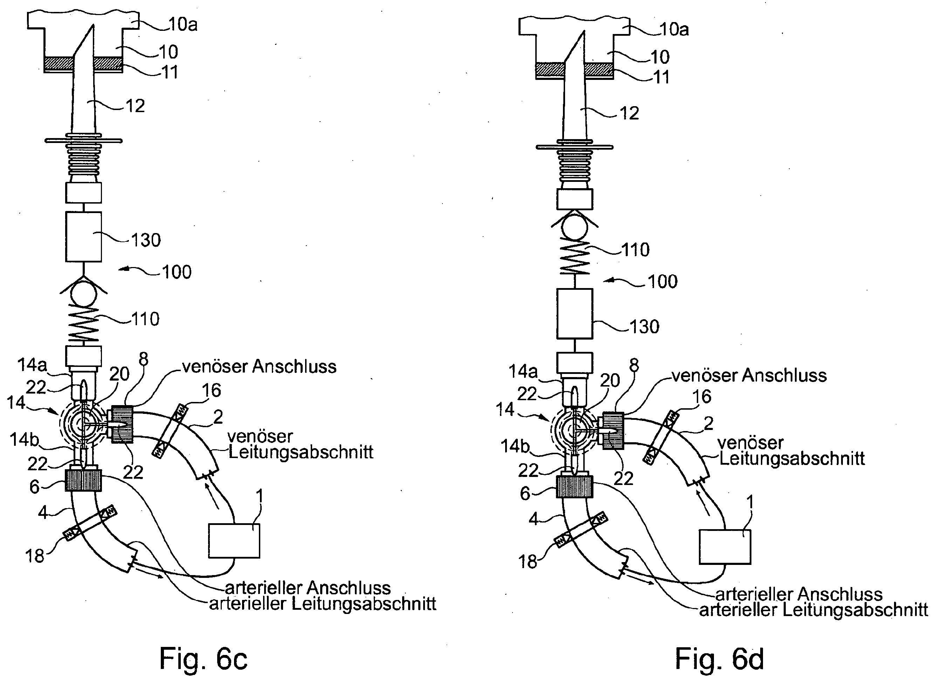

Alternativ hierzu ist es aber auch möglich das Rückschlagventil 110 gemäß der Fig. 6a mit einer Tropfkammer 130 zu kombinieren, indem das Rückschlagventil gemäß der Fig. 6c stromab zur Tropfkammer 130 oder gemäß der Fig. 6d stromauf zur Tropfkammer 130 angeordnet ist.

Dabei sei abschließend darauf hingewiesen, dass insbesondere das Rückschlagventil 110 ein gegenüber bekannten Vorrichtungen zusätzliches Bauteil darstellt, welches durch äußere Einwirkungen beschädigt oder abgerissen werden könnte. Um dies zu vermeiden bietet sich die Möglichkeit an, das Rückschlagventil 110 unmittelbar am Spike 12 und/oder am 3 -Wege - Hahn 14 anzuschließen oder in deren Anschlüsse zu integrieren, um dadurch eine Baueinheit auszubilden.

Die vorliegende Erfindung betrifft zusammenfassend eine Befüllvorrichtung beilspielsweise in Form einer Zirkulationsvorrichtung eines extrakorporalen Blutbehandlungsgeräts 1 mit einem vorzugsweise universellen medizinischen Flüssigkeitsbehälter 10, an den wahlweise ein arterieller Leitungsabschnitt 4 eines Fluidleitungssystems des extrakorporalen Blutbehandlungsgeräts 1 anschließbar ist. Die Befüllvorrichtung hat ferner eine Kontaminations-Verhinderungseinrichtung mit einem Spike sowie eine manuelle Fluid-Sperrvorrichtung beispielsweise eine 3 - Wege - Weiche, vorzugsweise einen 3 - Wege - Hahn 14, der dem Spike 12 (unmittelbar) nachgeordnet ist. Ein Weg 14a der 3 - Wege - Weiche ist mit dem Spike 12 oder dergleichen Anschlussmittel gekoppelt oder einstückig mit diesem ausgebildet. Zwischen dem Spike 12 und der manuellen Fluid-Sperrvorrichtung 14 ist ein Rückschlagventil 110 zwischengeschaltet, das so ausgerichtet ist, dass es eine Fluidströmung in Richtung zum Fluidbehälter 10 automatisch blockiert.

## Patentansprüche

1. Befüllvorrichtung eines Fluidleitungssystems eines extrakorporalen Blutbehandlungsgeräts (1) aufweisend:
eine Kontaminations-Verhinderungseinrichtung mit einem Spike (12) sowie einer manuell betätigbaren Fluid-Sperreinrichtung (14), die unmittelbar stromab zu dem Spike (12) angeordnet ist, wobei ein Rückschlagventil (110) zwischen Spike (12) und Fluid-Sperreinrichtung (14) vorgesehen ist, das dazu angepasst ist, nur eine Strömung vom Spike (12) in Richtung zur manuell betätigbaren Fluid-Sperreinrichtung (14) zuzulassen, wobei die manuell betätigbare Fluid-Sperreinrichtung (14) zumindest einen Fluidauslassanschluss hat, der dafür angepasst ist, dass ein arterieller Blutleitungsabschnitt (4) des Fluidleitungssystems des extrakorporalen Blutbehandlungsgeräts (1) im Betrieb der Befüllvorrichtung daran lösbar angeschlossen ist, **dadurch gekennzeichnet, dass**
die Fluid-Sperreinrichtung eine 3 - Wege - Weiche, vorzugsweise ein 3 - Wege - Hahn (14) ist, und
ein mittels des Spike (12) konnektierter Fluid- oder Flüssigkeitsbehälter (10) vorgesehen ist, der für einen Befüllvorgang nach dem single-pass-Prinzip an den arteriellen Blutleitungsabschnitt (4) des Fluidleitungssystems gekoppelt ist, indem die 3 - Wege - Weiche, vorzugsweise der 3 - Wege - Hahn (14) der Befüllvorrichtung in eine erste Schaltstellung gebracht wird, in welcher ein einen Fluideinlass bildender erster Weg (14a) mit einem zweiten Weg (14b) der 3 - Wege - Weiche, vorzugsweise des 3 - Wege - Hahns (14) fluidverbunden ist, an welchem der arterielle Blutleitungsabschnitt angeschlossen ist, wobei ein dritter Weg (14c) der 3 - Wege - Weiche, vorzugsweise des 3 - Wege - Hahns (14) gesperrt ist und
der arterielle Blutleitungsabschnitt (4) für einen Zirkulationsvorgang mit einem venösen Blutleitungsabschnitt (2) verbunden ist, indem die 3 - Wege - Weiche, vorzugsweise der 3 - Wege - Hahn (14) in eine zweite Schaltstellung gebracht wird, wobei in dieser zweiten Schaltstellung der erster Weg (14a) der 3 - Wege - Weiche, vorzugsweise des 3 - Wege - Hahns (14) gesperrt ist und damit der Fluid- oder Flüssigkeitsbehälter (10) vom Fluidleitungssystem des extrakorporalen Blutbehandlungsgeräts (10) fluidgetrennt ist, wohingegen der zweite Weg (14b) mit dem dritten Weg (14c) fluidverbunden ist, an den der venöse Blutleitungsabschnitt (2) angeschlossen ist und
in einer dritten Schaltstellung der 3 - Wege - Weiche, vorzugsweise des 3 - Wege - Hahns (14) dessen sämtliche Wege (14a - 14c) gesperrt sind, sodass in dieser Schaltstellung die beiden Blutleitungsabschnitte (2, 4) des Fluidleitungssystems von dem Flüssigkeitsbehälter (10) abklemmt sind.

2. Befüllvorrichtung nach Anspruch 1, **dadurch gekennzeichnet, dass** das Rückschlagventil (110) einen vom Fluid aus dem Fluidbehälter betätigbaren Ventilkörper hat, der mittels einer Feder, vorzugsweise einer Spiralfeder gegen die Strömungsrichtung aus dem Spike (12) auf einen Ventilsitz vorgespannt ist.

3. Befüllvorrichtung nach Anspruch 1 oder 2, **dadurch gekennzeichnet, dass** der Spike (12) und das Rückschlagventil (110) zu einer Einheit verbaut sind, indem das Rückschlagventil (110) vorzugsweise in einen Spikeanschluss integriert ist.

4. Befüllvorrichtung nach Anspruch 1 oder 2, **dadurch gekennzeichnet, dass** die manuell betätigbare Fluid-Sperreinrichtung (14) und das Rückschlagventil (110) zu einer Einheit verbaut sind, indem das Rückschlagventil (110) vorzugsweise in einen Einlass-Anschluss der manuell betätigbaren Fluid-Sperreinrichtung (14) integriert ist, an dem der Spike (12) angeschlossen ist.

5. Befüllvorrichtung nach einem der Ansprüche 1 bis 4, **dadurch gekennzeichnet, dass** der Spike (12), die manuell betätigbare Fluid-Sperreinrichtung (14) und das Rückschlagventil (110) zu einer Einheit integriert sind, bei der der Spike (12) unmittelbar ohne Zwischenleitung mit der manuell betätigbaren Fluid-Sperreinrichtung (14) gekoppelt ist und das Rückschlagventil (110) im Bereich der Koppelstelle angeordnet ist.

6. Befüllvorrichtung nach einem der Ansprüche 1 bis 4, **gekennzeichnet durch** ein Tropfkammer-Element, das stromab zum Spike (12) und vorzugsweise stromab zum Rückschlagventil (110) angeordnet ist und über eine Verbindungs-Schlauchleitung vorbestimmter Länge mit der manuell betätigbaren Fluid-Sperreinrichtung (14) verbunden ist.

7. Befüllvorrichtung nach Anspruch 1, **dadurch gekennzeichnet, dass** die Fluid-Sperreinrichtung ein manuell betätigbares Schließventil (14) ist.

8. Befüllvorrichtung nach Anspruch 1, **dadurch gekennzeichnet, dass** der 3 - Wege - Hahn (14) manuell betätigbar ist, wofür dieser einen mit einer Handhabe (22) ausgebildeten Drehkolben (20) aufweist.

9. Befüllvorrichtung nach einem der Ansprüche 1 bis 8, **dadurch gekennzeichnet, dass** an die Fluid-Sperrvorrichtung (14) der Spike (12) als Anschlussmittel (12) über einen Luer-Lock-Anschluss angeschlossen und/oder mit der Fluid-Sperrvorrichtung (14) zu einer integralen Baueinheit zusammengefasst ist.

10. Befüllvorrichtung nach Anspruch 9, **dadurch gekennzeichnet, dass** der Spike (12) übergangslos ohne Zwischenschaltung eines überbrückenden Leitungsstücks unmittelbar mit der Fluid-Sperrvorrichtung (14) verbunden ist.

11. Medizinisches Fluidleitungssystem eines Blutreinigungsgeräts vorzugsweise einer Dialysemaschine mit einem arteriellen und einem venösen Leitungsabschnitt, an deren Leitungsabschnittsenden jeweils ein Anschluss, vorzugsweise ein Luer-Lock-Anschuss vorgesehen ist, **gekennzeichnet durch** eine Befüllvorrichtung gemäß einem der vorstehenden Ansprüche 1 bis 10, an die zumindest der arterielle Blutleitungsabschnitt wahlweise für einen Befüllvorgang zumindest nach dem single-pass-Prinzip anschließbar ist.

12. Medizinisches Fluidleitungssystem nach Anspruch 11, **dadurch**
**gekennzeichnet, dass** zumindest der arterielle Blutleitungsabschnitt unmittelbar stromab zu seinem mit der Befüllvorrichtung zusammenwirkenden Anschluss eine Sperreinrichtung vorzugsweise eine Schlauchklemme aufweist, mittels welcher der arterielle Leitungsabschnitt temporär für ein Umklemmen von der Befülleinrichtung zu einem arteriellen Patientenzugang und umgekehrt fluidgesperrt wird.

13. Medizinisches Fluidleitungssystem nach Anspruch 11 oder 12, **dadurch**
**gekennzeichnet, dass** der venöse Blutleitungsabschnitt unmittelbar stromab zu seinem mit der Befüllvorrichtung zusammenwirkenden Anschluss eine Sperreinrichtung vorzugsweise eine Schlauchklemme aufweist, mittels welcher der venöse Leitungsabschnitt temporär für ein Umklemmen von der Befülleinrichtung zu einem arteriellen Patientenzugang und umgekehrt fluidgesperrt wird.

14. Verfahren zur Durchführung eines Befüll- und Rezirkulationsvorgangs eines blutseitigen Leistungssystems einer Dialysemaschine unter Verwendung einer Befüllvorrichtung nach einem der Ansprüche 1 bis 10, mit den Schritten:
a. Anschließen des Spike (12) an den einzigen Fluidanschluss des medizinischen Fluidbehälters (10) bei vollständig gesperrtem 3-Wege-Hahn (14) sowie Anschließen des arteriellen Leitungsabschnitts (4) an den zweiten Weg (14b) des noch gesperrten-3-Wege-Hahns (14) bei zur Umgebung hin geöffnetem venösem Fluidleitungsabschnitt (2),
b. Durchspülen eines Fluidströmungspfads entlang des arteriellen Fluidleitungsabschnitts (4) des extrakorporalen Blutbehandlungsgeräts (1) und des venösen Fluidleitungsabschnitts (2) durch Fluidverbinden des Spike (12) mit dem zweiten Weg (14b) des 3-Wege-Hahns (14),
c. vollständiges Sperren des 3-Wege-Hahns (14) und Anschließen des venösen Fluidleitungsabschnitts (2) an den dritten Weg (14c) des 3-Wege-Hahns (14),
d. Rezirkulieren des eingefüllten Fluids in dem Fluidströmungspfad durch Kurzschließen des zweiten und dritten Wegs (14b, 14c) am 3-Wege-Hahn bei gleichzeitigem Sperren des Spike (12),
e. vollständiges Sperren des 3-Wege-Hahns (14) und Abkoppeln des venösen Fluidleitungsabschnitts (2) vom dritten Weg (14c) sowie erneutes Durchspülen gemäß Schritt b. und
f. vollständiges Sperren des 3-Wege-Hahns als Vorbereitung für das darauffolgende Anschließen der befüllten arteriellen und venösen Fluidleitungsabschnitte (2, 4) an einen Patienten.

15. Verwendung einer Befüllvorrichtung nach einem der Ansprüche 1 bis 10 zur Befüllung eines blutseitigen Fluidleitungssystems einer Dialysemaschine vorzugsweise nach einem Verfahren gemäß Anspruch 14.

## Claims

1. Filling device of a fluid conducting system of an extracorporeal blood treatment device (1), comprising
an anti-contamination device with a spike (12) and a manually operable fluid blocking mechanism (14) which is arranged directly downstream of the spike (12), wherein a non-return valve (110) is provided between the spike (12) and the fluid blocking mechanism (14), wherein the non-return valve (110) is adapted to allow only a flow from the spike (12) in the direction to the manually operable fluid blocking mechanism (14), wherein the manually operable fluid blocking mechanism (14) has at least one fluid outlet connector which is adapted in such a way that an arterial blood line section (4) of the fluid conducting system of the extracorporeal blood treatment device (1) is connected to it in a detachable manner while the filling device is in operation, **characterized in that**
the fluid blocking mechanism is a 3-way switch, preferably a 3-way valve (14), and
a fluid or liquid container (10) connected via the spike (12) is provided, which is coupled with the arterial blood line section (4) of the fluid conducting system for the filling process based on the single-pass principle by putting the 3-way switch, preferably the 3-way valve (14), of the filling device in a first switch position, in which a first conduit (14a) constituting a fluid inlet is fluidically connected with a second conduit (14b) of the 3-way switch, preferably of the 3-way valve (14), to which the arterial line section is connected, wherein a third conduit (14c) of the 3-way switch, preferably of the 3-way valve (14) is closed, and
the arterial line section (4) is connected with a venous line section (2) for the circulation process by putting the 3-way switch, preferably the 3-way valve (14), in a second switch position, wherein in this second switch position, the first conduit (14a) of the 3-way switch, preferably the 3-way valve (14), is closed and thus the fluid container (10) is fluidically separated from the fluid conducting system of the extracorporeal blood treatment device (10), whereas the second conduit (14b) is fluidically connected with the third conduit (14c) to which the venous line section (2) is connected, and
in a third switch position of the 3-way switch, preferably the 3-way valve (14), all of its conduits (14a - 14c) are closed, so that in this switch position, the two line sections (2, 4) of the fluid conducting system are clamped from the fluid container (10).

2. Filling device according to claim 1, **characterised in that** the non-return valve (110) has a valve body that can be activated by the fluid from the fluid container, which is pre-tensioned on a valve seat by means of a spring, preferably a spiral spring, against the direction of flow from the spike (12).

3. Filling device according to claim 1 or 2, **characterised in that** the spike (12) and the non-return valve (110) are combined in a unit by integrating the non-return valve (110) preferably in a spike connector.

4. Filling device according to claim 1 or 2, **characterised in that** the manually operable fluid blocking mechanism (14) and the non-return valve (110) are combined in a unit by integrating the non-return valve (110) preferably in an inlet connector of the manually operable fluid blocking mechanism (14) on which the spike (12) is connected.

5. Filling device according to one of the claims 1 to 4, **characterised in that** the spike (12), the manually operable fluid blocking mechanism (14) and the non-return valve (110) are integrated in a unit in which the spike (12) is coupled directly without intermediate pipe with the manually operable fluid blocking mechanism (14), and the non-return valve (110) is arranged in the area of the coupling point.

6. Filling device according to one of the claims 1 to 4, **characterised by** a drip chamber element which is arranged downstream of the spike (12) and preferably downstream of the non-return valve (110) and is connected with the manually operable fluid blocking mechanism (14) through a connecting hose pipe of a predetermined length.

7. Filling device according to claim 1, **characterised in that** the fluid blocking mechanism is a manually operable closing valve (14).

8. Filling device according to claim 1, **characterised in that** the 3-way valve (14) can be activated manually, for which purpose it is equipped with a rotary piston (20) designed with a handle (22).

9. Filling device according to one of the claims 1 to 8, **characterised in that** on the fluid blocking mechanism (14), the spike is connected as a connecting device (12) with a Luer-Lock fitting, and/or is combined with the fluid blocking mechanism (14) into an integral modular unit.

10. Filling device according to claim 9, **characterised in that** the spike (12) is connected transition-free without interposition of a bridging line section directly with the fluid blocking mechanism (14).

11. Medical fluid conducting system of a blood purification device, preferably a dialysis machine, with an arterial line section and a venous line section, on whose line section ends one connector each, preferably a Luer-Lock fitting, is provided, **characterised by** a filling device according to one of the claims 1 to 10 above, to which at least the arterial blood line section can optionally be connected for a filling process at least on the basis of the single-pass principle.

12. Medical fluid conducting system according to claim 11, **characterised in that** at least the arterial blood line section has, directly downstream of its connector that interacts with the filling device, a blocking mechanism, preferably a hose clamp, by means of which the arterial line section is temporarily fluidically disconnected for reconnecting from the filling device to an arterial patient access and vice versa.

13. Medical fluid conducting system according to claim 11 or 12, **characterised in that** the venous blood line section has, directly downstream of its connector that interacts with the filling device, a blocking mechanism, preferably a hose clamp, by means of which the venous line section is temporarily fluidically disconnected for reconnecting from the filling device to an arterial patient access and vice versa.

14. Procedure for performing a filling and recirculation process of a blood-side conducting system of a dialysis machine using a filling device according to one of the claims 1 to 10, with the steps:
a. Connecting of the spike (12) to the single fluid connector of the medical fluid container (10) with the 3-way valve (14) completely closed, and connecting of the arterial line section (4) to the second conduit (14b) of the still closed 3-way valve (14) with the venous fluid line section (2) open to the environment,
b. Flushing of a fluid flow path along the arterial fluid line section (4) of the extracorporeal blood treatment device (1) and the venous fluid line section (2) by fluidically connecting the spike (12) with the second conduit (14b) of the 3-way valve (14),
c. Complete closing of the 3-way valve (14) and connecting of the venous fluid line section (2) to the third conduit (14c) of the 3-way valve (14),
d. Recirculating of the filled-in fluid in the fluid flow path by short-circuiting the second and third conduits (14b, 14c) on the 3-way valve with simultaneous closing of the spike (12),
e. Complete closing of the 3-way valve (14) and uncoupling of the venous fluid line section (2) from the third conduit (14c) and repeated flushing according to step b. and
f. Complete closing of the 3-way valve in preparation for the subsequent connecting of the filled arterial and venous fluid line sections (2, 4) to a patient.

15. Use of a filling device according to one of the claims 1 to 10 to fill a blood-side fluid conducting system of a dialysis machine, preferably based on a procedure according to claim 14.

## Revendications

1. Dispositif de remplissage d'un système de conduite de fluide d'un appareil de traitement du sang extracorporel (1) présentant :
un dispositif d'empêchement de contamination avec une pointe (12) ainsi qu'un dispositif de blocage de fluide (14) actionnable manuellement qui est agencé directement en aval de la pointe (12), un clapet antiretour (110) étant prévu entre la pointe (12) et le dispositif de blocage de fluide (14), lequel clapet est adapté afin d'autoriser seulement un écoulement de la pointe (12) en direction du dispositif de blocage de fluide actionnable manuellement (14), le dispositif de blocage de fluide actionnable manuellement (14) présentant au moins un raccord de sortie de fluide qui est adapté afin qu'une portion de conduite de sang (4) artérielle du système de conduite de fluide de l'appareil de traitement de sang extracorporel (1) soit raccordée en fonctionnement du dispositif de remplissage de manière amovible à celui-ci,
**caractérisé en ce que**
le dispositif de blocage de fluide est un dispositif de séparation à trois voies, de préférence un robinet à trois voies (14), et
un récipient de fluide ou de liquide (10) relié à l'aide de la pointe (12) est prévu, lequel est couplé pour un processus de remplissage selon le principe à une seule passe à la portion de conduite de sang artérielle (4) du système de conduite de fluide en ce que le dispositif de séparation à trois voies, de préférence le robinet à trois voies (14) du dispositif de remplissage est amené dans une première position de commutation, dans laquelle une première voie formant une entrée de fluide (14a) est reliée par voie fluidique à une deuxième voie (14b) du dispositif de séparation à trois voies, de préférence du robinet à trois voies (14), à laquelle la portion de conduite de sang artérielle est raccordée, une troisième voie (14c) du dispositif de séparation à trois voies, de préférence du robinet à trois voies (14), étant bloquée et
la portion de conduite de sang artérielle (4) pour un processus de circulation étant reliée à une portion de conduite de sang veineuse (2) **en ce que** le dispositif de séparation à trois voies, de préférence le robinet à trois voies (14), est amené dans une deuxième position de commutation, la première voie (14a) du dispositif de séparation à trois voies, de préférence du robinet à trois voies (14) étant bloquée dans cette deuxième position de commutation et le récipient de fluide ou de liquide (10) étant séparé en fluide du système de conduite de fluide de l'appareil de traitement de sang (10) extracorporel, alors que la deuxième voie (14b) est reliée en fluide à la troisième voie (14c), à laquelle la portion de conduite de sang (2) veineuse est raccordée et
dans une troisième position de commutation du dispositif de séparation à trois voies, de préférence du robinet à trois voies (14), l'ensemble de ses voies (14a-14c) étant bloqué de sorte que dans cette position de commutation, les deux portions de conduite de sang (2, 4) du système de conduite de fluide soient détachées du récipient de liquide (10).

2. Dispositif de remplissage selon la revendication 1, **caractérisé en ce que** le clapet antiretour (110) a un corps de clapet actionnable par du fluide provenant du récipient de fluide, lequel corps est précontraint à l'aide d'un ressort, de préférence un ressort hélicoïdal contre le sens d'écoulement depuis la pointe (12) sur un siège du clapet.

3. Dispositif de remplissage selon la revendication 1 ou 2, **caractérisé en ce que** la pointe (12) et le clapet antiretour (110) sont montés en une unité **en ce que** le clapet antiretour (110) est intégré de préférence dans un raccord de pointe.

4. Dispositif de remplissage selon la revendication 1 ou 2, **caractérisé en ce que** le dispositif de blocage de fluide actionnable manuellement (14) et le clapet antiretour (110) sont montés en une unité, **en ce que** le clapet antiretour (110) est intégré de préférence dans un raccord d'entrée du dispositif de blocage de fluide actionnable manuellement (14), auquel la pointe (12) est raccordée.

5. Dispositif de remplissage selon l'une quelconque des revendications 1 à 4, **caractérisé en ce que** la pointe (12), le dispositif de blocage de fluide actionnable manuellement (14) et le clapet antiretour (110) sont intégrés en une unité, pour laquelle la pointe (12) est couplée directement sans conduite intermédiaire avec le dispositif de blocage de fluide (14) actionnable manuellement et le clapet antiretour (110) est agencé dans la zone du point de couplage.

6. Dispositif de remplissage selon l'une quelconque des revendications 1 à 4, **caractérisé par** un élément de chambre compte-gouttes qui est agencé en aval de la pointe (12) et de préférence en aval du clapet antiretour (110) et est relié par une conduite de tuyau de liaison de longueur prédéterminée au dispositif de blocage de fluide (14) actionnable manuellement.

7. Dispositif de remplissage selon la revendication 1, **caractérisé en ce que** le dispositif de blocage de fluide est un clapet de fermeture (14) actionnable manuellement.

8. Dispositif de remplissage selon la revendication 1, **caractérisé en ce que** le robinet à trois voies (214) est actionnable manuellement, pour quoi celui-ci présente un piston rotatif (20) réalisé avec une manette (22).

9. Dispositif de remplissage selon l'une quelconque des revendications 1 à 8, **caractérisé en ce que** la pointe (12) est raccordée au dispositif de blocage de fluide (14) comme moyen de raccordement (12) par un raccord Luer Lock et/ou est réunie au dispositif de blocage de fluide (14) en une unité de construction intégrale.

10. Dispositif de remplissage selon la revendication 9, **caractérisé en ce que** la pointe (12) est reliée sans passage sans intercaler de pièce de conduite chevauchante directement au dispositif de blocage de fluide (14).

11. Système de conduite de fluide médical d'un appareil de nettoyage du sang de préférence d'une machine de dialyse avec une portion de conduite artérielle et une portion de conduite veineuse, auxquelles extrémités de portion de conduite est prévu respectivement un raccord, de préférence un raccord Luer Lock, **caractérisé par** un dispositif de remplissage selon l'une quelconque des revendications 1 à 10, auquel au moins la portion de conduite de sang artérielle peut être raccordée au choix pour un processus de remplissage au moins selon le principe à une seule passe.

12. Système de conduite de fluide médical selon la revendication 11, **caractérisé en ce qu'**au moins la portion de conduite de sang artérielle présente, directement en aval de son raccord coagissant avec le dispositif de remplissage, un dispositif de blocage, de préférence une pince pour tuyaux souples, à l'aide de laquelle la portion de conduite artérielle est bloquée en fluide temporairement pour un changement de raccordement du dispositif de remplissage à un accès de patient artériel et inversement.

13. Système de conduite de fluide médical selon la revendication 11 ou 12, **caractérisé en ce que** la portion de conduite de sang veineuse présente, directement en aval de son raccord coagissant avec le dispositif de remplissage, un dispositif de blocage, de préférence une pince pour tuyaux souples, à l'aide de laquelle la portion de conduite veineuse est bloquée en fluide temporairement pour un changement de raccordement du dispositif de remplissage à un accès de patient artériel et inversement.

14. Procédé de réalisation d'un processus de remplissage et recirculation d'un système de conduite côté sang d'une machine de dialyse en utilisant un dispositif de remplissage selon l'une quelconque des revendications 1 à 10, avec les étapes suivantes :
a. le raccordement de la pointe (12) à l'unique raccord de fluide du récipient de fluide médical (10) en cas de robinet à trois voies complètement bloqué (14) ainsi que le raccordement de la portion de conduite artérielle (4) à la deuxième voie (14b) du robinet à trois voies encore bloqué (14) en cas de portion de conduite de fluide (2) veineuse ouverte vers l'environnement,
b. le rinçage d'une voie d'écoulement de fluide le long de la portion de conduite de fluide artérielle (4) de l'appareil de traitement de sang extracorporel (1) et de la portion de conduite de fluide veineuse (2) par liaison de fluide de la pointe (12) à la deuxième voie (14b) du robinet à trois voies (14),
c. le blocage complet du robinet à trois voies (14) et le raccordement de la portion de conduite de fluide veineuse (2) à la troisième voie (14c) du robinet à trois voies (14),
d. la recirculation du fluide rempli dans la voie d'écoulement de fluide par court-circuitage de la deuxième et troisième voies (14b, 14c) sur le robinet à trois voies en cas de blocage simultané de la pointe (12),
e. le blocage complet du robinet à trois voies (14) et le découplage de la portion de conduite de fluide (2) veineuse de la troisième voie (14c) ainsi que le nouveau rinçage selon l'étape b. et
f. le blocage complet du robinet à trois voies comme préparation pour le raccordement consécutif des portions de conduite de fluide artérielle et veineuse remplies (2, 4) à un patient.

15. Utilisation d'un dispositif de remplissage selon l'une quelconque des revendications 1 à 10 pour le remplissage d'un système de conduite de fluide côté sang d'une machine de dialyse de préférence selon un procédé selon la revendication 14.
